(19) Europäisches Patentamt
European Patent Office
Office européen des brevets

(11) Publication number: **0 151 110**
**B1**

(12) **EUROPEAN PATENT SPECIFICATION**

(45) Date of publication of patent specification: **01.03.89**

(51) Int. Cl.$^4$: **C 07 D 211/60**

(21) Application number: **83902297.7**

(22) Date of filing: **01.08.83**

(86) International application number:
**PCT/NO83/00029**

(87) International publication number:
**WO 85/00599 14.02.85 Gazette 85/04**

(54) **L-N-n-PROPYLPIPECOLIC ACID-2,6-XYLIDIDE AND METHOD FOR PREPARING THE SAME.**

(43) Date of publication of application:
**14.08.85 Bulletin 85/33**

(45) Publication of the grant of the patent:
**01.03.89 Bulletin 89/09**

(84) Designated Contracting States:
**AT BE CH DE FR GB LI LU NL SE**

(56) References cited:
**DE-A-1 770 408**

**Acta pharmacologica et toxicologica, vol. 41, pp. 432-443, (1977)**

(73) Proprietor: **Astra Läkemedel Aktiebolag**
**Strängnäsvägen 44**
**S-151 85 Södertälje (SE)**

(72) Inventor: **THURESSON AF EKENSTAM, Bo**
**P.O. Box 93**
**S-440 74 Hjälteby (SE)**
Inventor: **BOVIN, Christer**
**Nils Kaggs gata 25**
**S-252 54 Helsingborg (SE)**

(74) Representative: **Danielsson, Sten Ove et al**
**AB ASTRA Patent and Trademark Department**
**S-151 85 Södertälje (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

A large variety of N-alkyl-pipecolic acid amides have been synthesized. A number of these compounds have found use as local anesthetics, such as Mepivacaine, namely the racemate of N-methylpipecolic-acid-2,6-xylidide:

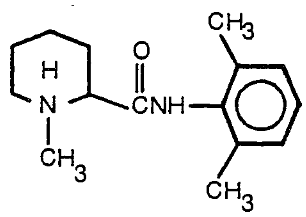

and Bupivacaine, namely the racemate of N-butylpipecolic acid-2,6-xylidide:

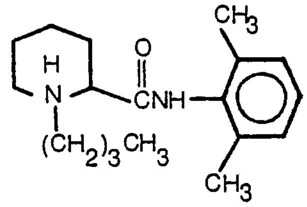

References disclosing homologs of this series of compounds include U.S. Patent 2,799,679; British Patent 775,749; British Patent 775,750; British Patent 800,565; British Patent 824,542; British Patent 869,978; British Patent 949,729; U.S. Patent 4,110,331; and U.S. Patent 4,302,465.

There is a summary paper dealing with these types of anesthetics, and related compounds in a paper in *Acta Chemica Scandinavica 11*, (1957) No. 7 pp. 1183—1190 by Bo Thuresson af Ekenstam et al.

There is a discussion of the effect of optical isomers in related compounds in *J. Med. Chem.*, 14 (1971) pp. 891—892 entitled "Optical Isomers Of Mepivacaine And Bupivacaine" by Benjamin F. Tullar; *Acta Pharm. Suecica*, 8 (1971) pp. 361—364 entitled "Some physicochemical Properties of the Racemates And The Optically Active Isomers Of Two Local Anaesthetic Compounds", by P. Friberger et al.; *Acta Pharmacol et Toxicol*, 31 (1972) pp. 273—286 entitled "Toxicological And Local Anaesthetic Effects Of Optically Active Isomers Of Two Local Anaesthetic Compounds", by G. Aberg; *Annual Review Of Pharmacology*, 9 (1969) pp. 503—520 entitled "Duration of Local Anaesthesia", by F. P. Luduena and *Acta Pharmacol. et Toxicol*, 41 (1977) pp. 432—443 entitled "Studies On The Duration of Local Anaesthesia: Structure/Activity Relationships In A Series Of Homologous Local Anaesthetics", by G. Alberg et al.

Summary Of The Invention

We have found that L-N-n-propylpipecolic acid-2,6-xylidide is markedly superior as a local anesthetic for mammals including humans to other known homologues of these compounds including the commercially exploited local anesthetics Mepivacaine, which is the N-methyl homolog, and Bupivacaine, which is the N-n-butyl homolog. This is surprising because the analgesic activity of the racemic n-propyl compound (R-N-n-propylpipecolic acid-2,6-xylidide) is so far below that of the racemic Bupivacaine (R-N-n-butylpipecolic acid-2,6-xylidide) as to stultify any further research on the n-propyl compound. Furthermore, research on the optical isomers of Mepivacaine and Bupivacaine reveal that the differences in potency compared to the racemate were not of such magnitude as to justify the commerical production of the optical isomers.

The effect of modifying the alkyl group on the nitrogen atom in the piperidine ring of this family of homologs is not completely understood. However, it is clear that any alkyl group of five or more atoms on this nitrogen atom is too toxic to function as a local anesthetic.

We have discovered that the laevo optical stereo-isomer N-n-propylpipecolic acid-2,6-xylidide has optimal properties as a local anesthetic.

Our method of preparing L-N-n-propylpipecolic acid-2,6-xylidide comprises resolving pipecolic acid to isolate the laevo optical stereoisomer. The laevo pipecolic acid hydrochloride is then chlorinated to form a laevo pipecolic acid chloride hydrochloride:

L– 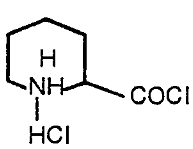

The acid chloride hydrochloride is then reacted with 2,6-xylidine to form the L-pipecolic acid-2,6-xylidide hydrochloride:

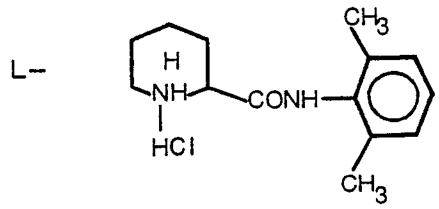

L-pipecolic acid-2,6-xylidide is then propylated to yield the L-N-n-propylpipecolic acid-2,6-xylidide.

Detailed Description Of The Preferred Embodiment

The following illustrative process described in four steps may be used to prepare the L-N-n-propylpipecolic acid-2,6-xylidide:

Example 1

Resolution of pipecolic acid

130 grams of pipecolic acid and 158.6 grams of Laevo (+)-tartaric acid are dissolved under stirring in 2,000 ml 95% ethyl alcohol and 125 ml water at a temperature of approximately 80°C. The solution is allowed to cool to room temperature and after two days the crystallized D-pipecolic-tartrate is separated. The L-pipecolic-tartrate remains in solution.

The filtrate is evaporated and dissolved in 5% acetic acid. Finally the solution is treated with Amberlite IR 45* in an ion exchanger. The eluate thus obtained is evaporated and the resulting crystalline residue is dried with potassium hydroxide in vacuo. The product obtained consists of L-pipecolic acid $(\alpha)_D^{24}$ −26.2° (C=5**, $H_2O$).

Example 2

Preparation of L-pipecolic acid chloride hydrochloride

The chlorination of the L-pipecolic acid hydrochloride is achieved by adding during a time period of 15—20 minutes four grams of phosphorus pentachloride to a suspension of four grams of L-pipecolic acid hydrochloride in 40 ml acetylchloride. The initial reaction is effected at a temperature of about 35°C under stirring for a time duration of two hours. The chlorination is completed by adding during a time period of about 10 minutes an additional two grams of phosphorus pentachloride and stirring over a further period of four hours while maintaining the suspension at a temperature of about 35°C.

The resulting L-pipecolic acid chloride hydrochloride is filtered and washed with toluene and acetone. The crystalline residue is then dried in vacuo. The product starts to sinter at 140°C (using a microscope) then decomposes at 150°C and all is melted at 155°C.

Example 3

Preparation of L-pipecolic acid-2,6-xylidide

A mixture of 2.7 ml 2,6-xylidine, 4 ml acetone, and 4 ml N-methylpyrrolidone is gradually added under stirring for two hours at a temperature of approximately 70°C to a suspension of 4 grams of L-pipecolic acid chloride hydrochloride in 30 ml of dry toluene. This yields a crystalline product, which is filtered, washed with acetone and dried. This crystalline product is then dissolved in water and the base is precipitated by the addition of ammonia.

The base is then extracted by the use of toluene and is removed by evaporation. The base is recrystallized from a mixture of hexane and ethanol to yield L-pipecolic acid-2,6-xylidide. The melting point of this compound is 129—130°C, $[\alpha]_D^{25}$ + 46.4° (C=1, 1 M HCl).

Example 4

Preparation of L-N-n-propylpipecolic acid — 2,6-xylidide

7.9 ml n-propylbromide and 6.8 grams of potassium carbonate are added to a solution of 17 grams of L-pipecolic acid-2,6-xylidide dissolved in 60 ml of isopropyl alcohol. Thereafter, 5 ml of water is added to the mixture and the reaction is carried out for a period of about four hours at approximately 72°C.

To complete the reaction, a further 0.8 ml n-propylbromide are added under continuous stirring and heating for 4 hours. Thereafter the solvent is carefully evaporated off under reduced pressure (20—25mm Hg). The residue is treated with a mixture of 250 ml toluene and an equal amount of water under gentle

*A commercial weakly basic polystyrene-polyamine anion exchange resin sold by Rohm & Haas Company of Philadelphia, Pa. designed primarily to remove strong acids.

**C means concentration in grams per 100 ml of solution.

heating at approximately 50°C. The toluene layer is separated and washed three times with 100 ml warm water (40°C). A 175 ml portion of the toluene is removed by evapoation and the remainder is stored in a refrigerator at +5°C for 6 hours to achieve crude crystalline L-N-n-propylpipecolic acid-2,6-xylidide.

The crystalline product is separated by filtration, washed with some cooled toluene and dried at 70°C.

Approximately 16 grams of crude L-N-n-propylpipecolic acid-2,6-xylidide are obtained. Recrystallization from toluene gives approximately 14 grams of the pure product, m.p. 144—146°C, $(\alpha)_D^{25}$ −82.0° (C=2, MeOH).

This product is dissolved in 100 ml ethanol and neutralized with concentrated hydrochloric acid.

Ethanol is removed by evaporation and the hydrochloride product obtained is vacuum dried. Finally the latter is recrystallized from 75 ml isopropyl alcohol. The yield is approximately 12 grams of L-N-n-propylpipecolic acid-2,6-xylidide hydrochloride, m.p. 260—262°C, $(\alpha)_D^{25}$ −6.6° (C=2, $H_2O$).

The reactions for achieving the compound of the present invention may be shown diagrammatically (commencing with L-pipecolic acid hydrochloride):

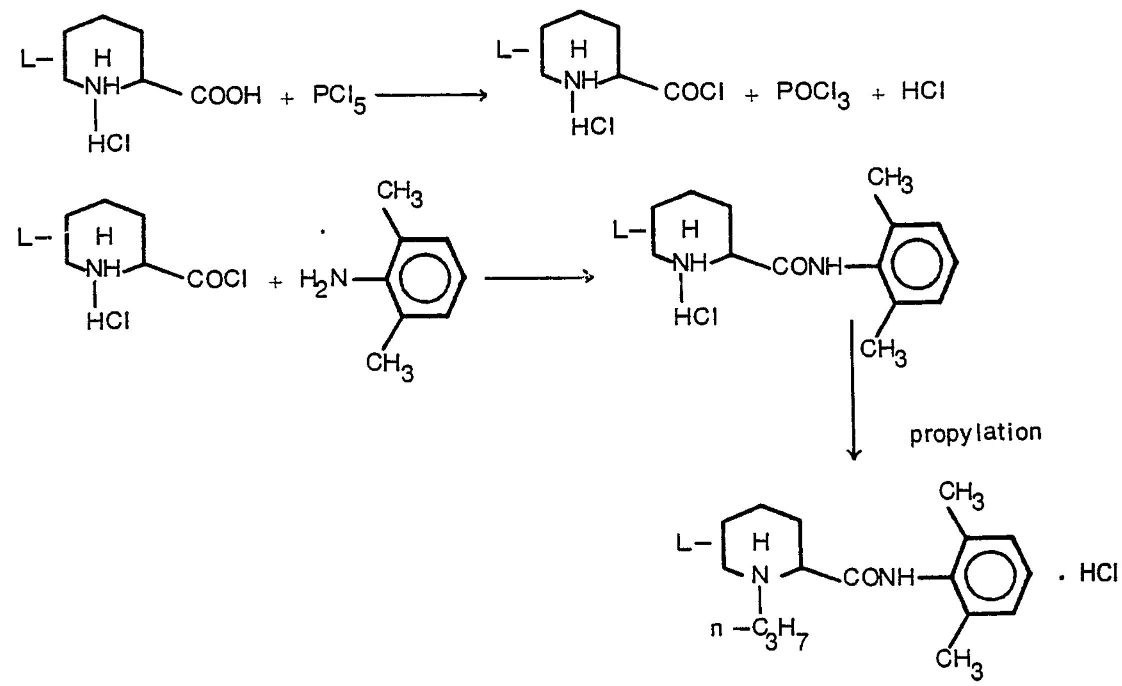


Useful background information concerning the synthesis of the homologs of N-alkyl-pipecolic acid-2,6-xylidide is contained in British Patents Nos. 775,750; 775,749; 824,542; 800,565 and 949,729; and in the article by Ekenstam et al. in *Acta Chem. Scand.* 11 (1957) No. 7, pp. 1183—1190, the disclosures of which are incorporated by reference.

A toxicological-pharmacological evaluation of the compound of the present invention, and relates homologs is set forth below.

The toxicity of the homologous series of N-alkyl-pipecolic acid-2,6-xylidides increases step wise and quickly with the lengthening of the alkyl chain. This is manifest from Table 1 set forth below, which compares racemic mixtures of alkyl homologues having from 1 to 5 carbon atoms.

TABLE 1
TOXICOLOGICAL DATA

| No. Structure (Racemate) | Molecular weight | Tissue* toxicity limits in % | $LD_{50}$** I.v. (intravenous) mg/kg/mouse |
|---|---|---|---|
| 1 N-methylpipecolic acid-2,6-xylidide | 246.46 | 3.5 | 40.3 |
| 2 N-ethylpipecolic acid-2,6-xylidide | 260.17 | 3.0 | 21.0 |
| 3 N-n-propylpipecolic acid-2,6-xylidide | 274.18 | 1.5 | 13.6 |
| 4 N-n-butylpipecolic acid-2,6-xylidide | 288.19 | 0.75 | 7.8 |
| 5 N-n-pentylpipecolic acid-2,6-xylidide | 302.20 | slightly soluble in water and tissue irritating*** | |

* Trypan blue test; Hoppe et al. (1950), *39*, 147—151 "Use Of Trypan Blue And Rabbit Eye Tests For Irritation",
J. Amer. Pharm. Ass. (Scient. Ed.), the values are approximate threshold values of tissue irritancy.
** $LD_{50}$, i.e. 50% mortality of mice.
*** Studies On The Duration Of Local Anaesthesia: Structure/Activity Relationships In A Series Of Homologous Local Anaesthetics, by G. Aberg, et al., Acta pharmacol. et toxicol., *41* (1977) pp. 432—443, Table 1 at p. 435.

From Table 1, it can be seen that the toxicological effects of the subject homologous series increases stepwise and quickly when the N-alkyl chain is lengthened, both with regards to tissue toxicity limits as well as intravenous toxicity.

The optical stereoisomers of the substances Nos. 1, 2 and 4 in Table 1 have earlier been prepared in pure form. These products have also earlier been shown to give insignificant differences with regards to the parameters which are of vital importance for the clinical use of an anesthetic product. See study which involves the discussed substances according to Table 1, by G. Aberg et al., "Studies on the Duration of Local Anesthesia: Structure/Activity Relationship in a Series of Homologous Local Anesthetics", Acta Pharmacol. et Toxicol. 41 (1977) pp. 432—443.

Some of the pure stereoisomers, whose structures are referred to in Table 2, have, on repeated occasions, been studied with regards to possible importance differences of their practical anesthesiological importance. Among others, substance No. 1 in Table 1, has been studied by G. Aberg: Studies on Mepivacaine and its Optically Active Isomers with Special Reference to Vasoactive Properties, Dept. of Pharmacology, School of Medicine, Linkoping, Sweden, 1972.

According to this work no great differences in effect can be expected between the different stereoisomers in the homologous group starting with Mepivacaine (the N-methyl compound).

The stereoosomers of the substances Nos. 1, 2 and 4, (Table 2) have been prepared earlier in a pure form but have shown no significant clinical differences in humans with respect to their use as local anesthetics. According to the invention, the L-isomer of substance No. 3 has now been prepared in a pure form and the results of studies on intracutaneous wheals as well as finger blockades in humans have shown that this compound has surprisingly good properties.

By use of intracutaneous wheals it is possible to measure the analgesic effect of an anesthetic on the periphera nerve ending of the epidermis.

This technique gives information about:
(1) the duration of anesthesia.
(2) Epidermal diffusion.
(3) Vascular effects.

TABLE 2
INTRACUTANEOUS WHEALS[1]

| Substance No. Form* | Molecular weight base | Melting points HCl-salts °C | Opt. activity System | $(\alpha)_D^{25}$,o | conc. % | Analgesia duration minutes |
|---|---|---|---|---|---|---|
| 1 L | 246.16 | 293—95 | C=5** | −63 | 0.5 | 68±11 |
| R | | 260—62 | MeOH | 0 | " | 65±9 |
| D | | 293—95 | | +63 | " | 53±8 |
| 2 L | 260.17 | 255 | C=2 | −70 | 0.25 | 76±11 |
| R | | 252—54 | MeOH | 0 | " | 63±13 |
| D | | 255 | | +63.5 | " | 58±11 |
| 3 L | 274.18 | 260—62 | C=2 | −82 | 0.25 | 220±23** |
| R | | 260.5 | MeOH | 0 | " | 84±15 |
| D | | 260—62 | | +82 | " | 81±14** |
| 4 L | 288.19 | 255—57 | C=2 | −80.9 | 0.25 | 76±10 |
| R | | 255—56 | MeOH | 0 | " | 118±15 |
| D | | 255—57 | | +80.9 | " | 75±11 |
| 5 L | | Not usable as slightly soluble in water, irritating | | | | |
| R | | | | | | |
| D | | | | | | |

[1] *Infiltration anesthesia on forearms of man.* The test solutions were injected intracutaneously on the dorsum of the forearms of 12 healthy volunteers, 20—25 years old. Three wheals were made on each arm in a rotating system in a double-blind fashion. The local anesthetic effect was tested using a pin-prick technique. the "duration of anesthesia" was defined as the time during which none of six pin-pricks into the wheel was felt. The method had been described by Dhuner et al. (1972). Dhuner, K. G., D. H. Lewis, A. Nyquist, D. Selander & E. Stig: Vascular effects of the isomers of mepivacaine, *Acta Anaest. Scand.* 1972, Supple. 48, 45—52.

* L=Laevo, R=Racemic, D=Dextro

** C equals grams per 100 ml of solution.

*** New isolated stereoisomers

TABLE 3
RESULTS OF THE FINGER BLOCKADE

| Substance No. Form | Concentration % | Time of onset minutes* | Analgesia-duration minutes | Frequency** |
|---|---|---|---|---|
| 1 L | 1.0 | 7±3 | 124±21 | 11/12 |
| R | " | 7±2 | 102±22 | 13/13 |
| D | " | 7±2 | 71±13 | 9/13 |
| 2 L | 1.5 | 9±2 | 141±18 | 11/13 |
| R | " | 10±1 | 123±19 | 12/12 |
| D | " | 9±3 | 112±21 | 10/13 |
| 3 L | 0.25 | 12±2 | 739±46*** | 6/7 |
| R | " | 10±1 | 263±31 | 9/10 |
| D | " | 14±3 | 175±21*** | 7/8 |
| 4 L | 0.25 | 13±3 | 259±37 | 13/20 |
| R | " | 9±2 | 388±50 | 15/20 |
| D | " | 15±4 | 243±38 | 14/20 |
| 5 | | Not usable too irritating against tissue | | |

[2] *Digital nerve blocks in man.* The test solutions were injected to block the ulnar volar nerves on the 2nd and 4th fingers of the same volunteers who has participated in the intracutaneous wheal tests described above. The tests were carried out in a double blind fashion in a rotating system, so that each solution was used with equal frequency on each of the blocked fingers. A total of four injections, 1.0 ml per injection, was done in each subject. One week later the procedure was repeated in the same subjects using the remaining test solutions. This method has been described by Dhuner et al. (1972) (for citation see Table 2 footnote 1). To measure the efficacy and duration of the blocks, the individual fingers were pricked rapidly 10 times with a needle algesimeter and the number of painful pricks noted. Each finger was tested every five minutes until complete recovery. The time of complete anesthesia has been called "duration of anesthesia".
* i.e. — the time from injection to complete analgesia
** i.e. — the number of successful anesthesias of total number of anesthesias performed (no anesthesia is possible if the ulnar volar nerves are not contacted by the anesthetic)
*** new isolated stereoisomers

When comparing the results of intracutaneous wheal (Table 2) and finger-blockade (Table 3) it can be seen that of the 12 optically active and inactive structures, substance No. 3, namely L-N-n-propylpipecolic acid-2,6-xylidide, gives the optimal increase of anesthetic effects. This unexpected increase in effect runs contra to what would be expected from the activity of prior stereoisomers.

Until now, racemic Bupivacaine, namely No. 4R, has been the compound having the largest clinical use in this series. During the past 15 years Bupivacaine has had a world wide increase in clinical use. As the above data discloses the L-N-n-propylpipecolic acid-2,6-xylidide is strikingly superior to Bupivacaine, being both a far better anesthetic and much less toxic.

In order to give a better illustration of the differences in effect, a series of various intracutaneous wheals has been investigated with regards to the skin temperature within the wheals compared to the skin temperature around the outside of the wheals during anesthesia. Based on this investigation, interesting differences in the temperatures can be observed.

Equipment used for the temperature measurements (experimental)

The given values have been registered 10 minutes after the intracutaneous injection, and thereafter the control values were immediately determined.

The means value of 6 wheals for each substance forms the basis for the values. The control values, however, represent the mean value obtained from 4 different sites measured outside the wheals 90 degrees apart and 2 centimeters from the wheal's center.

In the temperature measurements, an Oriel temperature apparatus† has been used. This is battery driven and is connected to a detector (diameter, 2,5 mm) and has an accuracy of ± 0.1°C. The temperature of the skin is measured by pressing the detector against the skin with a pressure of 100 grams. It is retained

† A widely used apparatus to measure skin temperature.

against the skin until a constant temperature is obtained.

In Table 4, $T_1$ is the measurement obtained at the center of the wheal, and $T_2$ is the average of four measurements taken 90 degrees apart outside of the wheal and within 2 centimeters of the wheal's center.

TABLE 4
TEMPERATURE MEASUREMENTS, ACCURACY: + 0.1°C

| No. | Substance as HCl-salt | Conc. % | $T_1$ °C Intracutaneous Wheal | $T_2$ °C Controls | °C Difference |
|---|---|---|---|---|---|
| 1 | Lidocaine | 1.0 | 30.2 | 29.3 | +0.9 |
| 2 | Lidocaine + adrenaline* 1:200000 | 1.0 | 27.8 | 28.9 | −1.1 |
| 3 | R-Bupivacaine | 0.25 | 31.5 | 32.1 | −0.6 |
| 4 | L-N-n-propylpipecolic acid-2,6-xylidide | 0.25 | 30.0 | 31.2 | −1.2 |
| 5 | R-N-n-propylpipecolic acid-2,6-xylidide | 0.25 | 33.1 | 33.2 | −0.1 |
| 6 | R-N-2-ethyl-pipecolic acid-2,6-xylidide | 1.0 | 31.2 | 30.7 | +0.5 |

* Adrenaline (epinephrine) is a vasoconstrictor which reduces the cross-section of the wheal region's blood vessels and retains the Lidocaine in the wheal region.

From Table 4 is is seen that example No. 4, namely L-N-n-propylpipecolic acid-2,6-xylidide surprisingly, lowers the temperature over its wheal to approximately the same extent as the combination of lidocaine with adrenaline (Table 4, example No. 2). This may be due to an effect on the blood vessels within the wheal similar to that of adrenalin. The racemic compound of example No. 5 showed practically no change in temperature.

Tables 1—4 demonstrate the connection between L-N-n-propylpipecolic acid-2,6-xylidide and the most important parameters in each table, e.g. optical stereoisomers, duration of analgesia in intracutaneous wheals, the analgesic duration of fingerblocks, and the maximization of temperature decrease. It is manifest that L-N-n-propylpipecolic acid-2,6-xylidide is the optimal anesthetic within this homologous series.

L-N-n-propylpipecolic acid-2,6-xylidide may be used as an injectable local anesthetic in the form of a water soluble salt. It may be used as the base in suppositories, or it may be used as a topical anesthetic by being blended into conventional solvents and carriers including thixotropic mixtures which form gels, or in a suspension, or it may be tableted in conjunction with conventional tableting materials.

Salts of L-N-n-propylpipecolic acid-2,6-xylidide may be made with the common mineral acids, aliphatic carboxylic acids, aromatic carboxylic acids and amino acids. Conventional safeguards must, of course, be used in respect of L-N-n-propylpipecolic acid-2,6-xylidide, such as the use of isotonic solutions when the anesthetic is employed as an injectable. Such isotonic solutions may be prepared from suitable salts, such as the water soluble chlorides of sodim, potassium, calcium and magnesium respectively, or the water soluble sulphates of sodium, potassium and magnesium.

Of course, the concentration of L-N-n-propylpipecolic acid-2,6-xylidide when administered as a local anesthetic will be regulated to avoid tissue irritation or toxic reaction effects. The regulation of the concentration of this anesthetic can be achieved by following conventional toxicity tests and protocols heretofore established for local anesthetics.

**Claims for the Contracting States: BE CH DE FR GB LI LU NL SE**

1. Therapeutically active N-alkyl-pipercolic acid-2,6-xylidide or water soluble salts thereof, characterized in that it is the L-isomer of N-n-propylpipecolic acid-2,6-xylidide.

2. Method of preparing a compound of claim 1, characterized in reacting the L-isomer of pipecolic acid-2,6-xylidide with a propylating agent.

3. Method according to claim 2, characterized in reacting L-pipecolic acid chloride hydrochloride with 2,6-xylidine and react the thus obtained L-pipecolic acid-2,6-xylidide with a propylating agent.

4. Method in accordance with the claims 2 or 3, characterized in that as propylating agent is used a propyl halogenide.

5. Method in accordance with the claim 4, characterized in the halogenating agent is propyl bromide.

6. The L-isomer of N-n-propylpipecolic acid-2,6-xylidide or water soluble salt thereof for use as a local anaesthetic.

**Claims for Contracting State: AT**

1. Method of preparing therapeutically active L-isomer of N-propyl-pipecolic acid-2,6-xylidide or water soluble salts thereof, characterized in reacting the L-isomer of pipecolic acid-2,6-xylidide with a propylating agent.

2. Method according to claim 1, characterized in reacting L-pipecolic acid chloride hydrochloride with 2,6-xylidine and react the thus obtained L-pipecolic acid-2,6-xylidide with a propylating agent.

3. Method in accordance with the claims 1 or 2, characterized in that as propylating agent is used a propyl halogenide.

4. Method in accordance with claim 3, characterized in that the halogenating agent is propyl bromide.

5. Use of the L-isomer of N-n-propyl-pipecolic acid-2,6-xylidide or a water-soluble salt thereof for the manufacture of a medicament with local anaesthetic effect.

**Patentansprüche für die Vertragsstaaten: BE CH DE FR GB LI LU NL SE**

1. Therapeutisch Aktives N-alkyl-pipekolinsäure-2,6-xylidid oder wasserlösliches Salz davon, dadurch gekennzeichnet, daß es das L-Isomere von N-n-Propylpipekolinsäure-2,6-xylidid ist.

2. Verfahren zur Herstellung einer Verbindung nach Anspruch 1, gekennzeichnet durch die Umsetzung des L-Isomeren von Pipekolinsäure-2,6-xylidid mit einem Propylierungsmittel.

3. Verfahren nach Anspruch 2, gekennzeichnet durch die Umsetzung von L-Pipekolinsäurechloridhydrochlorid mit 2,6-Xylidin und Umsetzung des so erhaltenen L-Pipekolinsäure-2,6-xylidids mit einem Propylierungsmittel.

4. Verfahren nach den Ansprüchen 2 oder 3, dadurch gekennzeichnet, daß als Propylierungsmittel ein Propylhalogenid verwendet wird.

5. Verfahren nach Anspruch 4, dadurch gekennzeichnet, daß das halogenierungsmittel Propylbromid ist.

6. Das L-Isomere von N-n-Propylpipekolinsäure-2,6-xylidid oder wasserlösliche Salz davon zur Verwendung als Lokalanaesthetikum.

**Patentansprüche für den Vertragsstaat: AT**

1. Verfahren zur Herstellung des therapeutisch aktiven L-Isomeren von N-Propyl-pipekolinsäure-2,6-xylidid oder wasserlöslicher Salze davon, gekennzeichnet durch die Umsetzung des L-Isomeren von Pipekolinsäure-2,6-xylidid mit einem Propylierungsmittel.

2. Verfahren nach Anspruch 1, gekennzeichnet durch die Umsetzung von L-Pipekolinsäurechloridhydrochlorid mit 2,6-Xylidin und Umsetzung des so erhaltenen L-Pipekolinsäure-2,6-xylidids mit einem Propylierungsmittel.

3. Verfahren nach den Ansprüchen 1 oder 2, dadurch gekennzeichnet, daß als Propylierungsmittel ein Propylhalogenid verwendet wird.

4. Verfahren nach Anspruch 3, dadurch gekennzeichnet, daß das Halogenierungsmittel Propylbromid ist.

5. Verwendung des L-Isomeren von N-n-Propylpipekolinsäure-2,6-xylidid oder eines Wasserlöslichen Salzes davon zur Herstellung eines Medikaments mit lokalanaesthetischer Wirkung.

**Revendications pour les Etats contractants: BE CH DE FR GB LI LU NL SE**

1. 2,6-xylidide d'acide N-alkylpipécolique thérapeutiquement actif ou ses sels hydrosolubles, caractérisé en ce qu'il l'isomère L du 2,6-xylidide d'acide N-n-propylpipécolique.

2. Procédé de préparation d'un composé selon la revendication 1, caractérisé en ce que l'on fait réagir l'isomère L du 2,5-xylidide d'acide pipécolique avec un agent de propylation.

3. Procédé selon la revendication 2, caractérisé en ce que l'on fait réagir le chlorhydrate de chlorure d'acide L-pipécolique avec de la 2,6-xylidine et que l'on fait réagir le 2,6-xylidide d'acide L-pipécolique ainsi obtenu avec un agent de propylation.

4. Procédé selon la revendication 2 ou 3, caractérisé en ce qu'on emploie un halogénure de propyle comme agent de propylation.

5. Procédé selon la revendication 4, caractérisé en ce que l'agent d'halogénation est le bromure de propyle.

6. L'isomère L du 2,6-xylidide d'acide N-n-propylpipécolique ou un de ses sels hydrosolubles utilisable comme anesthésique local.

**Revendications pour l'Etat contractant: AT**

1. Procédé de préparation de l'isomère L du 2,6-xylidide d'acide N-propylpipécolique

thérapeutiquement actif ou de ses sels hydrosolubles, caractérisé en ce que l'on fait réagir l'isomère L du 2,6-xylidide d'acide pipécolique avec un agent de propylation.

2. Procédé selon la revendication 1, caractérisé en ce que l'on fait réagir le chlorhydrate de chlorure d'acide L-pipécolique avec de la 2,6-xylidine et fait réagir le 2,6-xylidide d'acide L-pipécolique ainsi obtenu avec un agent de propylation.

3. Procédé selon la revendication 1 ou 2, caractérisé en ce qu'on emploie un halogénure de propyle comme agent de popylation.

4. Procédé selon la revendication 3, caractérisé en ce que l'agent d'halogénation est le bromure de propyle.

5. Utilisation de l'isomère L du 2,6 xylidide d'acide N-n-proylpipécolique ou d'un de ses sels hydrosolubles pour la fabrication d'un médicament doué d'une action anesthésique locale.